# EUROPÄISCHE PATENTANMELDUNG

(11) **EP 4 725 540 A1**
(43) Veröffentlichungstag der Anmeldung: **15.04.2026**
(21) Anmeldenummer: 24206150.5
(22) Anmeldetag: 11.10.2024
(51) Int. Cl.: A61M 60/178, A61M 60/232, A61M 60/422, A61M 60/822, A61M 60/855

(54) **PUMPE, INSBESONDERE BLUTPUMPE, UND BESTIMMUNG IHRES DIFFERENZDRUCKS**

(71) Anmelder: Berlin Heart GmbH, 12247 Berlin (DE); Medizinische Universität Wien, 1090 Wien (AT)
(72) Erfinder: PETERS, Oliver, 12247 Berlin (DE); GRANEGGER, Marcus, 2380 Perchtoldsdorf (AT); ABART, Theodor, 2340 Mödling (AT)
(74) Vertreter: Pfenning, Meinig & Partner mbB

(57) **Zusammenfassung**

Die Erfindung bezieht sich auf eine Pumpe, insbesondere Blutpumpe, umfassend: eine Pumpenkammer (2), in der ein um eine Rotationsachse (3) drehbar gelagerter Rotor (1) mit wenigstens einem Förderelement (1a, 1b) zur Förderung von einer Flüssigkeit, insbesondere Blut, in einer Radialrichtung oder mit einer Radialrichtungskomponente angeordnet ist, eine Wandung (4), die die Pumpenkammer umgibt, wobei die Pumpenkammer einen umfangsseitigen Pumpenauslass aufweist, einen Stator mit mehreren Antriebsspulen (5, 6, 21) zur Erzeugung eines Antriebsmagnetfeldes, das dazu eingerichtet ist, eine Rotationsbewegung des Rotors anzutreiben sowie eine Steuereinrichtung (7), die dazu eingerichtet ist, Antriebsströme in den Antriebsspulen für den Rotationsantrieb des Rotors zu erzeugen, wobei der Rotor ein wenigstens teilweise elektrisch leitendes Target (18, 18') in Form eines Körpers aufweist, der von Mess-Wechselmagnetfeldern durchsetzbar ist, und wobei wenigstens ein Empfangsspulenpaar zum Empfang der Mess- Wechselmagnetfelder derart gestaltet ist, dass eine der Empfangsspulen den Abstand zum Target mit höherer Empfindlichkeit nachweist als die andere Empfangsspule. Dadurch und durch ein entsprechendes Verfahren wird es möglich, mit dem Empfangsspulenpaar eine Rotorneigung und mit dieser zusammenhängende Messgrößen zu ermitteln.

## Beschreibung

Die Erfindung liegt auf dem Gebiet der Medizintechnik und umfasst Aspekte des Maschinenbaus und der Messtechnik sowie der Hydrodynamik. Bevorzugte Anwendung kann die Erfindung im Bereich der Blutpumpen, beispielsweise für Herzunterstützungssysteme, finden.

Viele Pumpenbauarten weisen üblicherweise antreibbare Rotoren auf, die Förderelemente für die zu fördernde Flüssigkeiten aufweisen oder tragen. Die Rotoren kommen mit den zu fördernden Flüssigkeiten in Berührung und unterliegen Kräften, die von der Pumpenlast abhängig sind.

Beispielsweise ist aus der europäischen Patentschrift EP2507613 B1 eine Blutpumpe mit einem Rotor bekannt.

Aus dem Dokument US2022/0409878 A1 sind bereits verschiedene Maßnahmen und Methoden zur Positionsmessung eines Rotors in einer Blutpumpe bekannt.

Vor dem Hintergrund des Standes der Technik liegt der vorliegenden Erfindung die Aufgabe zugrunde, eine Pumpe mit einem Rotor derart zu gestalten, dass in einfacher Weise die Ermittlung von Zustandsgrößen der Pumpe ermöglicht ist.

Die Aufgabe wird durch die Erfindung mit den Merkmalen der unabhängigen Patentansprüche gelöst. Die abhängigen Patentansprüche stellen mögliche Implementierungen vor.

Die Erfindung bezieht sich unter anderem auf eine Pumpe, insbesondere Blutpumpe, umfassend
eine Pumpenkammer, in der ein um eine Rotationsachse drehbar gelagerter Rotor mit wenigstens einem Förderelement zur Förderung von einer Flüssigkeit, insbesondere Blut, in einer Radialrichtung oder mit einer Radialrichtungskomponente angeordnet ist,
eine Wandung, die die Pumpenkammer umgibt, wobei die Pumpenkammer einen umfangsseitigen Pumpenauslass aufweist,
einen Stator mit mehreren Antriebsspulen zur Erzeugung eines Antriebsmagnetfeldes, das dazu eingerichtet ist, eine Rotationsbewegung des Rotors anzutreiben sowie
eine Steuereinrichtung, die dazu eingerichtet ist, Antriebsströme in den Antriebsspulen für den Rotationsantrieb des Rotors zu erzeugen,
wobei der Rotor ein wenigstens teilweise elektrisch leitendes Target in Form eines Körpers aufweist, der von Mess-Wechselmagnetfeldern durchsetzbar ist, die durch eine Wechselmagnetfeldquelle, insbesondere eine oder mehrere Erregungsspulen erzeugt werden, wobei die Pumpe ein oder mehrere Empfangsspulenpaare mit jeweils einander bezüglich der Rotationsachse gegenüberliegenden Empfangsspulen aufweist, in denen durch die Mess- Wechselmagnetfelder Wechselströme und/oder Wechselspannungen erzeugt werden, die von der Wechselwirkung der Empfangsspulen mit dem Target abhängig sind, wobei die Steuereinrichtung dazu eingerichtet ist, die Wechselströme und/oder Wechselspannungen in den Empfangsspulen zu messen und aus diesen komplexe Kenngrößen, insbesondere komplexe Widerstände von Empfangsspulen zu ermitteln, wobei wenigstens ein Empfangsspulenpaar oder die Pumpe derart gestaltet ist, dass eine der Empfangsspulen den Abstand zum Target mit höherer Empfindlichkeit nachweist als die andere Empfangsspule und/oder dass im Falle der Erzeugung der Mess-Wechselmagnetfelder durch eine oder mehrere Erregungsspulen diese dazu eingerichtet sind, im Bereich einer der Empfangsspulen eines Empfangsspulenpaares ein stärkeres Mess-Wechselmagnetfeld zu erzeugen als in dem Bereich der bezüglich der Rotationsachse gegenüberliegenden Empfangsspule, und dass die Steuereinrichtung dazu eingerichtet ist, aus den in einem Empfangsspulenpaar gemessenen Mess-Wechselspannungen und/oder Mess-Wechselströmen komplexe Empfangsspulen-Zustandsgrößen, insbesondere komplexe Empfangsspulen-Widerstände, des wenigstens einen Empfangsspulenpaares zu ermitteln und mit diesen Zustandsgrößen einen Differenzdruck und/oder eine Durchflussrate der Pumpe und/oder einen Neigungsgrad des Rotors und/oder eine Radialverschiebung des Rotors zu ermitteln.

Bei einer Pumpe der oben beschriebenen Art gemäß der Erfindung, bei der ein um eine Rotationsachse drehbar gelagerter Rotor in einer Pumpenkammer rotiert, die beispielsweise eine den Rotor umgebende, zylindersymmetrische oder nicht zylindersymmetrische Volute mit einem umfangsseitigen Pumpenauslass aufweist, wirken durch eine Brechung der Axialsymmetrie der Flüssigkeitsströmung im Pumpengehäuse im Betrieb während der Förderung einer Flüssigkeit Kräfte auf den Rotor, die nicht axialsymmetrisch bezüglich der Rotorachse sind und die Radialkomponenten aufweisen. Die auf den Rotor wirkenden Kräfte sind unter anderem abhängig von der Durchflussrate der Pumpe, dem Differenzdruck der Pumpe, der Viskosität der geförderten Flüssigkeit und in gewissem Maß auch von der Lage der Pumpe relativ zum Erdgravitationsfeld. Der Rotor ist in radialer und axialer Richtung in der Pumpe gelagert, wobei die Lagerung grundsätzlich einerseits mechanisches Spiel und andererseits auch eine elastische Nachgiebigkeit aufweist. Gelingt es, einen Grad einer Rotorneigung und/oder eine Radialverschiebung des Rotors zu ermitteln, so kann daraus unter anderem der Differenzdruck und/oder die Durchflussrate der Pumpe oder eine Kombination, insbesondere eine Linearkombination, aus diesen beiden Größen bestimmt oder geschätzt werden. Die Bestimmung der Rotorneigung und/oder der Radialverschiebung des Rotors kann aber auch für sich noch aus anderen Gründen sinnvoll sein, beispielsweise, um die Lager zu überprüfen, zu steuern oder zu regeln.

Grundsätzlich ist es bekannt, eine Rotorposition des Rotors einer Flüssigkeitspumpe durch die Wechselwirkung eines mit dem Rotor verbundenen Targets mit Empfangsspulen zu ermitteln, wobei in Empfangsspulen durch Mess-Wechselmagnetfelder Wechselströme und Wechselspannungen erzeugt werden, die von der Wechselwirkung der Empfangsspulen mit dem Target, insbesondere von dem Abstand der Empfangsspulen zum Target, abhängig sind.

Dabei sind in vielen Fällen Empfangsspulen ringförmig um die Rotationsachse herum verteilt, so dass eine Positionsmessung des Rotors in seiner Axialrichtung unabhängig mittels mehrerer Empfangsspulen möglich ist. Oft sind bei derartigen Messungen mehrere Empfangsspulen miteinander verbunden. Tritt in einem solchen Fall eine Verkippung/Neigung des Rotors ein, so können die dadurch eintretenden Änderungen der durch die Empfangsspulen erfassten Messgrößen sich ganz oder teilweise kompensieren, da eine Neigung des Rotors auf der einen Seite der Rotationsachse eine Annäherung des Targets an eine Empfangsspule und gleichzeitig auf der gegenüberliegenden Seite der Rotationsachse eine Entfernung des Targets von einer weiteren Empfangsspule mit sich bringt.

Insbesondere für eine Schweberegelung der Magnetlager, die den Rotor in axialer Richtung positionieren, wird ein Messwert der Axialposition des Rotors benötigt, die in der der Kraftrichtung der Aktoren der aktiven Lagerregelung gemessen wird und unempfindlich gegenüber Bewegungen in anderen Freiheitsgraden ist. Dies kann durch eine Mittelung der Messwerte mehrerer, einander diametral bezüglich der Rotorachse gegenüberliegender Sensoren erfolgen.

Um die genannten Kompensationen der Messwerte bei einer Rotorneigung zu vermeiden oder zu reduzieren, ist gemäß der Erfindung vorgesehen, dass bei

Empfangsspulenpaaren mit jeweils einander bezüglich der Rotationsachse gegenüberliegenden Empfangsspulen eine der Empfangsspulen den Abstand zum Target mit höherer Empfindlichkeit nachweist als die andere Empfangsspule. Damit ist auch bei einer Bewegung der Rotorachse im Sinne einer Neigung oder Radialverschiebung gewährleistet, dass sich Änderungen der Messgrößen, beispielsweise Änderungen der gemessene Abstände der beiden Empfangsspulen eines Empfangsspulenpaares vom Target oder von diesen Abständen abhängiger Messgrößen, nicht kompensieren und eine resultierende Messgröße eine Bestimmung dieser Änderungen ermöglicht.

Dies ist insbesondere dann der Fall, wenn jeweils die beiden Empfangsspulen eines Empfangsspulenpaares miteinander elektrisch verbunden, beispielsweise elektrisch in Reihe geschaltet, und gemeinsam mit einer Messeinrichtung zur Messung der Messgrößen verbunden sind. Eine aktuelle Axialposition des Rotors kann mit einem Empfangsspulenpaar oder besser noch mit mehreren weiteren Empfangsspulenpaaren, die in verschiedenen Umfangspositionen am Umfang des Rotors verteilt sind, durch Mittelwertbildung der Messwerte ermittelt werden und der Einfluss der veränderlichen Axialposition auf die Messung der Neigung und/oder Radialverschiebung des Rotors kann dann rechnerisch kompensiert werden.

Es kann auch vorgesehen sein, dass im Falle der Erzeugung der Mess-Wechselmagnetfelder durch eine oder mehrere Erregungsspulen diese dazu eingerichtet sind, im Bereich einer der Empfangsspulen eines Empfangsspulenpaares ein stärkeres Mess- Wechselmagnetfeld zu erzeugen als in dem Bereich der bezüglich der Rotationsachse gegenüberliegenden Empfangsspule. Auch bei dieser Realisierungsform ergibt sich im Fall einer Neigung oder Radialverschiebung des Rotors eine resultierende, unkompensierte Änderung einer Messgröße.

Weiter kann es auch vorgesehen sein, durch unterschiedliche Permeabilitäten in den Empfangsspulen oder in ihrer Umgebung verschiedene Nachweisempfindlichkeiten für die Mess- Wechselmagnetfelder zu erzeugen. Dies kann beispielsweise auch durch unterschiedliche Gestaltung der Pumpenwand im Bereich der verschiedenen Empfangsspulen erreicht werden, indem beispielsweise das Material der Pumpenwand lokal variiert wird oder ein zusätzlicher Materialbelag im Beriech einer der Empfangsspulen auf der Pumpenwand vorgesehen wird.

Eine besondere Ausführung kann vorsehen, dass die Steuereinrichtung dazu eingerichtet ist, den Differenzdruck und/oder eine Durchflussrate der Pumpe mit ermittelten Empfangsspulen-Zustandsgrößen des Rotors sowie einem oder mehreren der folgenden Messgrößen und/oder ihren zeitlichen Ableitungen zu ermitteln, insbesondere mittels eines trainierten selbstlernenden Systems oder eines Beobachters, der mit den Messgrößen ein die Messgrößen und mathematische Ableitungen der Messgrößen beschreibendes Differentialgleichungssystem durch ein numerisches Verfahren löst, oder eines in einer Speichereinrichtung hinterlegten Kennlinienfeldes:
Änderungsgeschwindigkeit der Empfangsspulen-Zustandsgrößen,
zweite zeitliche Ableitung der Empfangsspulen-Zustandsgrößen, Umdrehungsgeschwindigkeit des Rotors,
Leistung des Rotorantriebs,
Stromstärke des Antriebsstroms,
Axialposition des Rotors,
Ausrichtung der Pumpe relativ zum Erdgravitationsfeld,
lineare, auf die Pumpe wirkende Beschleunigung sowie
   auf die Pumpe wirkende Drehbeschleunigung.

Die auf den Rotor während des Förderbetriebs der Pumpe wirkenden Kräfte umfassen Beschleunigungskräfte, die durch die zweite zeitliche Ableitung der Empfangsspulen-Zustandsgrößen berücksichtigt werden können sowie Reibungskräfte, die von der Bewegungsgeschwindigkeit des Rotors, unter anderem in der Neigungsrichtung, und von der Viskosität der geförderten Flüssigkeit abhängen und durch die Änderungsgeschwindigkeit der Empfangsspulen-Zustandsgrößen abgebildet werden können. Zudem hängen die Kräfte von der Umdrehungsgeschwindigkeit des Rotors, der Leistung des Rotorantriebs, und der Stromstärke des Antriebsstroms ab. Da diese Größen oft in einer bestimmten Abhängigkeit zueinander stehen, kann es sinnvoll sein, eine oder auch mehrere dieser Größen bei der Ermittlung des Differenzdrucks der Pumpe mit einzubeziehen.

Da die Differenzialgleichungen, die die genannten Größen miteinander in Beziehung setzen, bekannt sind, lassen sich aus den Messwerten, sofern diese erfasst werden, durch einen sogenannten Beobachter, der Differenzialgleichungssysteme näherungsweise/numerisch lösen kann, alle Größen angeben oder zumindest schätzen. Dadurch lassen sich, falls sich aus den Messwerten der durch die Mess- Wechselmagnetfelder erzeugten Wechselströme und/oder Wechselspannungen eine Kombination aus dem Radialfluss der Pumpe und dem Differentialdruck ergibt, diese beiden Größen auch unabhängig voneinander berechnen.

Die lineare, auf die Pumpe wirkende Beschleunigung sowie
auf die Pumpe wirkende Drehbeschleunigung können mittels Lage- und Beschleunigungssensoren, beispielsweise linearer Beschleunigungssensoren zur Messung einer linearen oder Gyroskopen zur Messung einer Drehbeschleunigung an der Pumpe, beispielsweise am Pumpengehäuse, gemessen werden. Mit Beschleunigungs- und/oder Lagesensoren kann beispielsweise die Orientierung der Pumpe relativ zur Richtung der Erdanziehungskraft oder relativ zur Richtung von wirkenden Trägheitskräften bestimmt werden.

Da die Rotorneigung und/oder eine Radialverschiebung des Rotors aufgrund seines Gewichtes auch von der Wirkrichtung des Erdanziehungskraft abhängt, kann durch Berücksichtigung der Lage des Rotors /der Pumpe, genauer der Orientierung relativ zur Erdanziehungskraft, der Einfluss des Eigengewichts des Rotors unter dem Einfluss der Gravitation kompensiert werden. Kann der Differenzdruck und/oder die Durchflussrate und die Rotorneigung/Radialverschiebung ohne Berücksichtigung der Lage der Pumpe genügend genau bestimmt werden, so kann hierüber umgekehrt auch eine Lageerkennung der Pumpe realisiert werden.

Können mehrere der genannten Größen erfasst werden, so kann der ermittelte Differenzdruck der Pumpe mittels eines in einer Steuereinrichtung gespeicherten Kennlinienfeldes genauer bestimmt werden. Es kann auch eine selbstlernende Einrichtung, beispielsweise in Form eines neuronalen Netzes, mit Messwerten einer Auswahl der genannten Messgrößen und gleichzeitig bestimmten Differenzdruckwerten trainiert und danach zu einer genaueren Bestimmung des Differenzdrucks der Pumpe verwendet werden.

Es kann auch vorgesehen sein, dass die Pumpe am Umfang ihres Stators verteilt mehrere Empfangsspulenpaare von einander bezüglich der Rotationsachse gegenüberliegenden Empfangsspulen aufweist, und die Steuereinrichtung dazu eingerichtet ist, die Empfangsspulen-Zustandsgrößen mehrerer Paare von Empfangsspulen zu ermitteln und diese insbesondere miteinander zur Ermittlung eines Differenzdrucks der Pumpe zu verknüpfen.

Beim Pumpbetrieb können die auf den Rotor wirkenden Kräfte in Radialrichtung, die zu einer Neigung und/oder Radialverschiebung führen, jeweils durch ein Empfangsspulenpaar etwa in der Richtung einer Verbindungslinie der beiden Empfangsspulen des Paares erfasst werden. Eine Neigung oder Verschiebung des Rotors wird in der Richtung dieser Verbindungslinie gemessen. Senkrecht auf dieser Verbindungslinie erfolgende Verschiebungen werden nicht oder nur in geringem Maß erfasst. Aus diesem Grund kann es sinnvoll sein, beispielsweise ein Empfangsspulenpaar so auszuwählen, dass seine Verbindungslinie parallel zu einer zu erwartenden Neigungsrichtung oder Verschiebungsrichtung des Rotors liegt. Es können aber auch verschiedene Empfangsspulenpaare so angeordnet werden, dass sie die Neigung oder Verschiebung des Rotors in verschiedenen Richtungen erfassen. Die Sensitivität dieser unterschiedlich ausgerichteten Empfangsspulenpaare für Neigungen und Verschiebungen des Rotors in unterschiedlichen Richtungen ist unterschiedlich groß, so dass durch eine Auswahl von geeignet ausgerichteten Empfangsspulenpaaren oder eine Verknüpfung der durch sie erfassten Messwerte eine optimale Messempfindlichkeit erreicht werden kann.

Grundsätzlich werden die von den Empfangspumpenpaaren erfassten Messgrößen auch durch den Abstand des Targets und des Rotors von dem Empfangsspulen in Axialrichtung des Rotors beeinflusst. Werden mehrere Empfangsspulenpaare am Umfang des Rotors verteilt betrieben, so kann durch eine Mittelung ihrer Messwerte auch der Axialabstand des Targets und des Rotors und somit seine Axialposition ermittelt und geregelt sowie bei den übrigen Messungen berücksichtigt werden.

Eine weitere Ausführungsform kann konkreter vorsehen, dass die Pumpe am Umfang ihres Stators verteilt mehrere Paare von einander bezüglich der Rotationsachse gegenüberliegenden Empfangsspulen aufweist, und die Steuereinrichtung dazu eingerichtet ist, die Empfangsspulen-Zustandsgrößen mehrerer Paare von Empfangsspulen zu ermitteln und eine Neigung und/oder Radialverschiebung des Rotors in einer ersten Ebene zu ermitteln, die die Rotorachse enthält, sowie die Neigung und/oder Radialverschiebung in einer zu dieser Ebene um die Rotorachse gedreht liegenden zweiten Ebene und dass die Steuereinrichtung weiter dazu eingerichtet ist, mit der Neigung und/oder Radialverschiebung des Rotors in der ersten Ebene oder einer Kombination der Neigungen und/oder Radialverschiebungen in der ersten Ebene und der zweiten Ebene den Differenzdruck und/oder die Durchflussrate der Pumpe zu ermitteln.

Die verschiedenen Ebenen, in denen die Rotorneigung gemessen wird, können dabei beispielsweise um 30, 90 oder 120 Grad gegeneinander gedreht sein, was konstruktive Vorteile haben kann, jedoch grundsätzlich auch um beliebige andere Winkel.

Dabei kann es der Fall sein, dass die Neigung und/oder Radialverschiebung des Rotors in der Richtung in einer Ebene, die die Rotorachse und den Pumpenauslass enthält, am größten ist. Deshalb kann es sinnvoll sein, in dieser Richtung oder allgemein in einer Richtung, in der die Neigung des Rotors im Betrieb am stärksten ist, ein erstes Empfangsspulenpaar auszurichten, um eine hohe Sensitivität zu erreichen. Für andere Empfangsspulenpaare, die eine hiervon abweichende Ausrichtung ihrer Verbindungslinie haben, kann die Abhängigkeit der Messgrößen von der Neigung/Verschiebung des Rotors einen anderen Charakter haben und beispielsweise in Messbereichen sensitiv sein, in denen die Messung mit dem ersten Empfangsspulenpaar weniger sensitiv ist.

Es kann zudem vorgesehen sein, dass die Pumpe einen Absolutdrucksensor und/oder einen Magnetfeldsensor, insbesondere einen Hallsensor aufweist.

Mit Beschleunigungs- oder Lagesensoren kann beispielsweise die Orientierung der Pumpe relativ zur Richtung der Erdanziehungskraft bestimmt werden. Mit dem Absolutdrucksensor, der beispielsweise am Pumpeneinlass angeordnet sein kann, kann der Ventrikeldruck und nach Bestimmung des Differenzdrucks der Pumpe beispielsweise auch der Aortendruck bestimmt werden.

Es kann auch ein Magnetfeldsensor, beispielsweise ein Hall-Sensor vorgesehen sein, der eine Bestimmung der Rotationswinkelposition des Rotors erlaubt und somit eine Möglichkeit für ein schleiffreies Anlaufen des Pumpenrotors ermöglicht.

Der Motor kann grundsätzlich gestützt auf Messdaten von Magnetfeldsensoren kommutiert werden. Es ist jedoch auch denkbar, die Pumpe grundsätzlich über ein Back-EMF- Verfahren zu kommutieren und mittels Magnetfeldsensoren einen redundanten Winkelgeber bereitzustellen.

Magnetfeldsensoren sind ebenso wie die Wirbelstromsensoren in der Lage eine Rotorverkippung zu messen. Da diese aber das mehrpolige Magnetfeld des Rotors messen, ist hier dem kleinen Differenzsignal der Rotorverschiebung ein großes Signal der Magnetpole überlagert. Kleine Asymmetrien zwischen den Rotor-Magnetpolen können dann fälschlicherweise wie eine Rotorbewegung erscheinen.

Weiter kann vorgesehen sein, dass das Target als ein fest mit dem Rotor verbundener Körper, insbesondere als Scheibe oder als Ring, ausgebildet ist, der mit dem Rotor um dessen Rotationsachse rotiert und insbesondere in azimutaler Richtung bezüglich der Rotorachse betrachtet einen oder mehrere erste Bereiche mit einer ersten elektrischen Leitfähigkeit und einen oder mehrere zweite Bereiche mit einer von der ersten elektrischen Leitfähigkeit verschiedenen zweiten elektrischen Leitfähigkeit aufweist.

Grundsätzlich und für alle beschriebenen Ausführungsformen kann vorgesehen sein, dass das Target nicht homogen bezüglich der Rotationsachse ausgebildet ist, insbesondere wenigstens eine nicht zentrische Ausnehmung aufweist, die sich in der Umfangsrichtung um die Rotationsachse herum über einen begrenzten Winkelbereich erstreckt. Das Target kann ebenfalls, um einen ähnlichen Effekt zu erzielen, wenigstens einen nicht zentrischen Bereich aufweisen, in dem die elektrische Leitfähigkeit geringer ist, als in den übrigen Bereichen. Dies kann beispielsweise dadurch realisiert sein, dass das Material oder die Materialzusammensetzung sich in den genannten nicht zentrischen Bereichen von dem Material oder der Materialzusammensetzung in den übrigen Bereichen des Targets unterscheidet. Beispielsweise kann das Target aus Kupfer bestehen und Ausnehmungen aufweisen oder das Target kann insgesamt kreisscheibenförmig sein und neben Bereichen aus Kupfer, aus einem anderen Metall oder aus einem elektrisch leitenden oder mit Metallpartikeln gefüllten Kunststoff weitere Bereiche aufweisen, die aus einem Kunststoff mit geringer Leitfähigkeit oder einem sonstigen Isolierwerkstoff bestehen.

In diesem Fall ändert sich gemäß den obigen Ausführungen der Einfluss, den das Target auf jede der für die Abstandsmessungen verwendeten Empfangsspulen und Empfangsspulenpaare hat, periodisch mit der Rotation des Targets/Rotors relativ zu den Empfangsspulen. Bei Verwendung eines derartigen nicht homogenen Targets lässt sich somit durch die Auswertung der erfassten Dämpfung der Induktivitäten der Empfangsspulen neben der Axialposition oder der Radialposition und der Rotorneigung auch die Drehzahl und die Rotationswinkelposition des Rotors jederzeit ermitteln.

Eine weitere Ausführungsform kann vorsehen, dass das Target die Form einer ebenen, mit dem Rotor fest verbundenen Scheibe aufweist, deren Flächennormale in Richtung zu einer oder mehreren Empfangsspulen orientiert ist und dass der durch die Antriebsspulen erzeugte magnetische Fluss des Motors im Target überwiegend in Axialrichtung des Rotors verläuft oder dass das Target die Form eines mit dem Rotor fest verbundenen, am äußeren Umfang des Rotors angeordneten Torus aufweist und dass der durch die am Umfang des Pumpengehäuses angeordneten Antriebsspulen erzeugte magnetische Fluss des Motors im Target überwiegend in Radialrichtung des Rotors verläuft.

Es handelt sich in dem ersten genannten Fall um einen Axialflussmotor. Bei einem derartigen Axialflussmotor wird dabei in vielen Fällen eine planparallele, beispielsweise kreisrunde oder ringförmige Scheibe oder Platte als Target verwendet werden.

Ist das Target nicht gegenüber der Rotationsachse geneigt, so rotiert es mit dem Pumpenrotor in einem gleichmäßigen Abstand von den beispielsweise symmetrisch um die Rotationsachse angeordneten Empfangsspulen, so dass bei einem ideal rotationssymmetrischen Target alle Empfangsspulen bei der Rotation des Targets ständig demselben Einfluss des Targets unterliegen. Dieser hängt dann im Wesentlichen von einer veränderlichen Axialposition des Targets und somit des Rotors ab, die aus den in den Empfangsspulen erzeugten Signalen ermittelt werden kann.

In dem zweiten genannten Fall handelt es sich um einen Radialflussmotor. In diesem Fall können Antriebsspulen an der Umfangsseite des Rotors außerhalb der Pumpenwand verteilt sein, die das Antriebsfeld erzeugen. Das Target kann dann die Form eines Torus, oder eines Rohrabschnitts haben und mit dem Rotor auf dessen äußerer Umfangsseite fest verbunden sein. Die Antriebsspulen können wenigstens teilweise als Erregungs- und/oder Empfangsspulen ausgebildet sein. Sie reagieren auf eine radiale Auslenkung des Targets mit dem Rotor jeweils an der Axialposition der Empfangsspulen, die durch eine Rotorneigung verursacht ist.

Es kann in einer weiteren Gestaltung vorgesehen sein, dass das Target die Empfangsspulen eines Empfangsspulenpaares nicht vollständig überdeckt oder wenigstens während des Betriebs der Pumpe nicht vollständig überdeckt. Die Empfangsspulen wenigstens eines Empfangsspulenpaares können beispielsweise radial über das Target hinausragen.

In diesem Fall wird eine Radialverschiebung des Targets und des Rotors besonders sensitiv erfasst, da bei einer solchen Verschiebung zwar die Überdeckung mit einer ersten Empfangsspule eines Empfangsspulenpaares abnimmt und die Überdeckung mit der gegenüberliegenden zweiten Empfangsspule im gleichen Maß zunimmt, jedoch die erste und die zweite Empfangsspule diese Änderung mit unterschiedlicher Sensitivität nachweisen.

Eine weitere Ausgestaltung einer Pumpe der oben beschriebenen Art kann vorsehen, dass die Empfangsspulenpaare auf der Außenseite der Wandung der Pumpenkammer angeordnet sind und dass die Wandung der Pumpenkammer mehr als 25% des Mess-Wechselmagnetfeldes transmittiert und das Target mehr als 25% des Mess-Wechselmagnetfeldes reflektiert oder absorbiert und/oder dass die Wandung der Pumpenkammer aus einem ersten Material und das Target überwiegend aus einem zweiten Material besteht, wobei das zweite Material bei der Frequenz der Mess-Wechselspannung einen geringeren elektrischen Widerstand aufweist als das erste Material und wobei insbesondere das erste Material Titan oder eine Titanlegierung ist und/oder das zweite Material Kupfer oder eine Kupferlegierung.

Grundsätzlich können sowohl das erste Material als auch das zweite Material ein Metall oder ein nicht metallisches, elektrisch besser oder schlechter leitendes Material sein. Die Beeinflussung der Dämpfung oder der Induktivität von außerhalb des Pumpenraums angeordneten Empfangsspulen durch das Target, das sich in dem Pumpenraum auf dem Rotor befindet, wirkt bei geeigneter Wahl der Materialien und der Frequenz der gegebenenfalls durch eine Wechselmagnetfeldquelle, insbesondere eine oder mehrere Erregungsspulen erzeugten Mess-Wechselspannung auch durch das Material der Wandung des Pumpenraums hindurch. Der Fall von außerhalb des Pumpenraums befindlichen Empfangsspulen umfasst beispielsweise die Ausführungsform, bei der die Antriebsspulen als Empfangsspulen genutzt werden. Die Voraussetzung für eine erfolgreiche Materialwahl ist, dass bei der Frequenz der Mess-Wechselspannung im Material des Targets deutlich stärkere Wirbelströme erzeugt werden als in dem Material der Wandung des Pumpenraums. Zu diesem Zweck kann, falls die Wandung des Pumpenraums aus einer Titanlegierung besteht, wie sie bei medizinischen Implantaten häufig verwendet wird, das Target vorteilhaft beispielsweise aus Kupfer oder einer Kupferlegierung bestehen. Die Pumpenraumwandung kann jedoch ebenso aus einem nicht elektrisch leitenden Kunststoff bestehen. Die in der Pumpenraumwandung erzeugten Wirbelströme wirken bei den Messungen der Induktivitätsänderungen der Spulen als Messfehler und werden auf diese Weise möglichst gering gehalten. Die Wirbelströme in der Wandung schirmen das Target von der Empfangsspule, falls diese außerhalb des Pumpenraums angeordnet ist, ab und erzeugen somit sowohl einen Widerstandsoffset als auch eine Verringerung der Sensitivität. Diese Wirbelströme sind daher entweder klein oder in allen Phasen, das heißt in allen Empfangsspulen, möglichst gleich zu halten.

Zudem kann bei der Pumpe vorgesehen sein, dass die Steuereinrichtung dazu eingerichtet ist, in einer oder mehreren der Erregungsspulen jeweils periodische Mess-Wechselspannungen mit einer Frequenz zwischen 20kHz und 2MHz, insbesondere zwischen 50kHz und 100 kHz, zu erzeugen.

Dieser Frequenzbereich ist besonders bei einer Pumpenraumwandung aus Titan oder einer Titanlegierung und einem Target aus Kupfer vorteilhaft, weil in diesem Frequenzbereich die im Kupfer erzeugten Wirbelströme ausreichend groß und die in dem Titanwerkstoff erzeugten Wirbelströme deutlich kleiner sind.

Es kann bei der Pumpe auch vorgesehen sein, dass eine, mehrere oder alle Empfangsspulen und/oder Erregungsspulen mit Antriebsspulen des Stators der Pumpe identisch oder mit diesen verbunden sind.

Als Erregungsspulen zum Erzeugen eines Magnetfeldes, das mit dem Target des Rotors wechselwirkt, können somit einige oder alle Antriebsspulen dienen. Diese Antriebsspulen können alternativ oder zusätzlich auch wenigstens teilweise als Empfangsspulen dienen. Dies hat den Vorteil, dass keine zusätzlichen Spulen oder Anschlüsse eingebaut werden müssen und somit die Pumpe und die Driveline, das heißt die Verbindung der Pumpe mit einer Steuereinheit, kompakter ausgeführt werden können. Dies hat weiterhin den Vorteil, dass die Bedingungen der Erzeugung des oder der Magnetfelder einschließlich der Geometrie genau bekannt sind und dass die Mess-Wechselspannungen, die an den einzelnen Antriebsspulen anliegen, passend gestaltet werden können.

Die Antriebsspulen müssen nicht als einzelne Spule ausgeführt sein, sondern können auf mehrere in Reihe oder parallel geschaltete Spulen aufgeteilt sein. Diese Situation tritt insbesondere bei Poolpaarzahlen auf, die größer als 1 sind. In diesem Fall können die einzelnen Spulen, die jeweils als Teile der Antriebsspulen oder Antriebswicklungen elektrisch in Reihe geschaltet sind, jeweils auch Spulen eines Empfangsspulenpaares bilden.

Eine weitere Gestaltung der Pumpe kann vorsehen, dass jeweils eine der Empfangsspulen eines Empfangsspulenpaares , insbesondere im Fall, dass die Empfangsspulen mit Antriebsspulen des Stators der Pumpe identisch sind, eine der Antriebsspulen eines Paares, mit einem oder mehreren Elementen eines Schwingkreises, insbesondere mit einem Kondensator, verbunden ist und/oder dass in den beiden Empfangsspulen eines Empfangsspulenpaares oder in deren Umgebung Elemente mit verschiedener magnetischer Permeabilität angeordnet sind.

Eine solche Beschaltung einer der Empfangsspulen eines Empfangsspulenpaares stellt eine einfache Maßnahme dar, um die Sensitivität der beiden Empfangsspulen für die Messung von Mess-Wechselmagnetfeldern und für die Messung eines Abstands zum Target unterschiedlich zu gestalten. Nach Auslegung des Schwingkreises, beispielsweise als Parallel- oder Serienschwingkreis, kann eine Spule eines Empfangsspulenpaares sensitiver oder unempfindlicher ausgestaltet werden als die gegenüberliegende, zweite Spule des Empfangsspulenpaares. Es können auch Schwingkreise an beiden gegenüberliegenden Empfangsspulen eines Empfangsspulenpaares angeordnet werden. Hierbei kann dann beispielsweise eine Spule bei der Wirbelstromfrequenz, das heißt bei der Frequenz des Mess- Wechselmagnetfeldes, in Serienresonanz und die andere Spule in Parallelresonanz betrieben werden, um eine besonders hohe Empfindlichkeit bezüglich der Rotorneigung und/oder der Radialverschiebung zu erhalten.

Zudem oder als Alternativlösung können, wie weiter oben bereits beschrieben, auch die beiden Empfangsspulen eines Empfangsspulenpaares mit unterschiedlichen magnetischen Permeabilitäten kombiniert/versehen werden, beispielsweise dadurch, dass Körper verschiedener Permeabilität in die Empfangsspulen eingebracht oder in ihre Nähe gebracht oder auf der Pumpenwandung, beispielsweise in Form einer Materialschicht, angebracht werden.

Die Erfindung bezieht sich außer auf eine Pumpe der oben beschriebenen Art auch auf ein Verfahren zur Bestimmung des Differenzdrucks einer Pumpe, insbesondere einer Blutpumpe, die
eine Pumpenkammer, in der ein um eine Rotationsachse drehbar gelagerter Rotor mit wenigstens einem Förderelement zur Förderung von einer Flüssigkeit, insbesondere Blut, in einer Radialrichtung oder in einer Richtung mit einer Radialrichtungskomponente angeordnet ist,
eine Wandung, die die Pumpenkammer umgibt, wobei die Pumpenkammer einen umfangsseitigen Pumpenauslass aufweist,
einen Stator mit mehreren Antriebsspulen zur Erzeugung eines Antriebsmagnetfeldes, das dazu eingerichtet ist, eine Rotationsbewegung des Rotors anzutreiben sowie
eine Steuereinrichtung umfasst, die dazu eingerichtet ist, Antriebsströme in den Antriebsspulen für den Rotationsantrieb des Rotors zu erzeugen, wobei während des Betriebs der Pumpe eine Neigung und/oder Radialverschiebung der Rotorachse oder eine von diesen abhängige elektrische Größe und/oder zeitliche Ableitungen dieser Größen ermittelt wird/werden und mit einer oder mehreren dieser Größen ein Differenzdruck und/oder eine Durchflussrate der Pumpe ermittelt wird.

Weiter oben ist bereits im Zusammenhang mit der Beschreibung der erfindungsgemäßen Pumpe und ihrer Ausführungsformen ein Verfahren beschrieben worden, dass die Ermittlung der Neigung und/oder der Radialverschiebung der Rotorachse ermöglicht. Alternativ oder zusätzlich dazu kann die Neigung der Rotorachse auch beispielsweise durch mehrere im Bereich des Umfangs des Rotors angeordnete Hall-Sensoren ermittelt werden, die an mehreren Stellen des Rotorumfangs die Axialposition des Rotors bestimmen, um danach aus der Rotorneigung den Differenzdruck zu bestimmen. Dieses Verfahren kann in der Genauigkeit ausreichen, es ist jedoch grundsätzlich mit größeren Ungenauigkeiten verbunden als das oben beschriebene Verfahren, da die Magnetfeldmessung durch das Drehfeld der Antriebsspulen gestört wird.

Bei einem Verfahren der genannten Art kann auch vorgesehen sein, dass während der Ermittlung der Neigung und/oder Radialverschiebung der Rotorachse oder einer von diesen abhängigen elektrischen Größe mittels eines Lagesensors die Lage der Pumpe relativ zum Gravitationsfeld der Erde und insbesondere eine lineare Beschleunigung und/oder eine Winkelbeschleunigung der Pumpe ermittelt und bei der Ermittlung des Differenzdrucks und/oder der Durchflussrate als Korrekturgröße berücksichtigt wird/werden.

Mit der linearen Beschleunigung und der Winkelbeschleunigung der Pumpe ist in diesem Zusammenhang die Beschleunigung der gesamten Pumpe, nicht die Beschleunigung oder Winkelbeschleunigung des Rotors gemeint. Diese Grö-βen, nämlich die Beschleunigung und/oder Winkelbeschleunigung des Rotors, können zusätzlich und unabhängig davon durch weitere Sensoren, oder auch sensorlos über ein auf Gegeninduktion (back-EMF) basiertes Verfahren, ermittelt und berücksichtigt werden.

Im Folgenden wird die Erfindung anhand von Ausführungsbeispielen in Figuren einer Zeichnung gezeigt und nachfolgend beschrieben.

Dabei zeigt
Figur 1 schematisch eine Blutpumpe in einem Längsschnitt mit einem Rotor, einem Pumpenraum und Antriebsspulen,
Figur 2 schematisch eine Antriebsspule und ihre Wechselwirkung mit einem Target,
Figur 3 eine Blutpumpe in schematischer Darstellung mit einem Rotor ohne eine Neigung der Rotorachse,
Figur 4, eine Blutpumpe in schematischer Darstellung mit einem Rotor, dessen Rotorachse geneigt ist,
Figur 5 drei Empfangsspulenpaare mit jeweils einer Empfangsspule mit einer Kondensatorbeschaltung,
Figur 6 eine Ansicht von Antriebsspulen und einem kreisringförmigen Target in Axialrichtung bezüglich der Rotationsachse,
Figur 7 die Abhängigkeit der Rotorneigung in zwei verschiedenen Richtungen vom Differenzdruck der Pumpe,
Figur 8 die Abhängigkeit zwischen dem Drehmoment des Rotors und der Durchflussrate der Pumpe,
Figur 9 die Abhängigkeiten der Rotorneigung in einer ersten Richtung und in einer zweiten Richtung vom Differenzdruck der Pumpe für verschiedene Ausrichtungen der Pumpe,
Figur 10 das Frequenzverhalten verschiedener Materialen mit unterschiedlichen Eindringtiefen in Bezug auf Wirbelströme,
Figur 11 ein Phasenanschlussdiagramm eines Pumpenmotors bei der Verwendung von drei Antriebsspulen für die Messung, sowie
Figur 12 eine Ansicht von Antriebsspulen und einem kreisscheibenförmigen Target in Axialrichtung bezüglich der Rotationsachse, wobei das Target an seinem Umfang Ausnehmungen aufweist, die die Messung der Rotationsgeschwindigkeit ermöglichen.

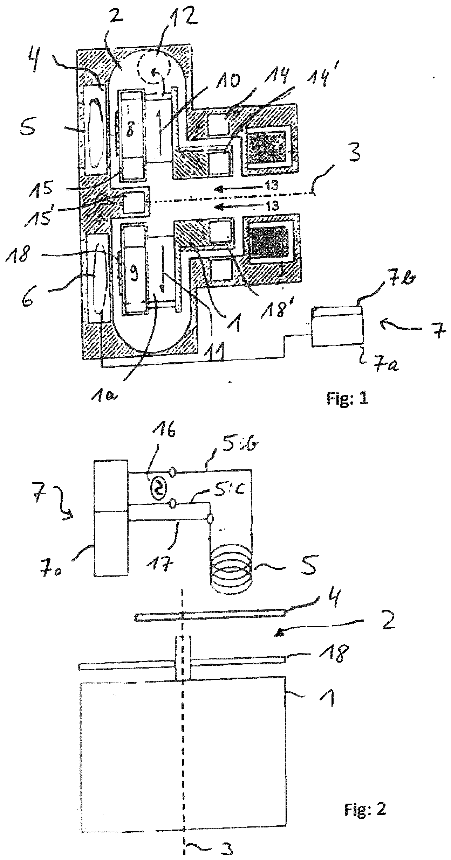

Figur 1 zeigt in einer Querschnittsansicht schematisch eine implantierbare Blutpumpe mit einem Rotor 1 der innerhalb einer Pumpenkammer 2 rotierend um eine Rotationsachse 3 gelagert ist. Durch eine Wandung 4 von der Pumpenkammer 2 getrennt sind außerhalb des Pumpenraums um die Rotationsachse 3 als Elemente eines Stators Antriebsspulen 5, 6 verteilt, die von einer Steuereinrichtung 7 angesteuert werden. Die Antriebsspulen 5, 6 erzeugen Magnetfelder, durch die mittels der Rotormagnete 8, 9 die Rotationsbewegung und in dem speziellen dargestellten Fall eine Axialbewegung des Rotors 1 angetrieben und/oder gesteuert wird.

Die zu fördernde Flüssigkeit, in dem konkret vorliegenden Fall Blut, wird entlang der Rotationsachse 3 in Richtung der Pfeile 13 zugeführt und durch die Rotationsbewegung des Rotors mit wenigstens teilweise in Radialrichtung verlaufenden Förderelementen 1a, 1b und die in diesen erzeugten Zentrifugalkräfte in Richtung der Pfeile 10, 11 in die Radialrichtung umgelenkt. Das Blut fließt dann über den Pumpenauslass in Form der umfangsseitigen Öffnung 12 in der Wandung 4 der Pumpenkammer/des Pumpengehäuses ab. Zudem sind in der Figur 1 magnetische Lager 14, 14' und 15, 15' dargestellt, die als Radiallager oder als kombinierte Radial/Axiallager ausgeführt sein können.

Die Steuereinrichtung 7 kann außer den Antriebsströmen zusätzlich die Mess-Wechselspannungen und Mess-Wechselströme erzeugen, die für die Ermittlung der komplexen Zustandsgrößen, insbesondere Widerstandswerte der Antriebsspulen verwendet werden, die auch als Empfangsspulen verwendet werden. Die Antriebsspulen 5, 6 bilden somit in diesem Fall sowohl die Erregungsspulen als auch die Empfangsspulen. Ein Target 18 in Form einer Scheibe kann auf den Rotormagneten 8, 9 zwischen diesen und den Antriebsspulen 5, 6 angeordnet werden. Weiter kann die Steuereinrichtung 7 die Mess- Wechselspannungen und/oder Mess-Wechselströme in den Antriebsspulen erfassen und auswerten und aus diesen einzeln oder aus den ermittelten komplexen Widerstandswerten oder einer alternativen Verknüpfung der gemessenen Strom- und Spannungsverläufe, beispielsweise unter Berücksichtigung der Phasenverschiebungen zwischen Strom- und Spannungsverläufen, eine Position des Targets und damit des Rotors in Axialrichtung ermitteln. Die Steuereinrichtung 7 weist neben einer Auswerteeinheit 7a auch eine Mess-Kontrollvorrichtung 7b zur regelmäßigen Überprüfung und/oder Plausibilitätsprüfung der Messwerte auf, die dazu eingerichtet ist, fehlerhafte Spulen zu detektieren. Die Mess- Kontrollvorrichtung kann zu diesem Zweck einen Mikrocontroller oder einen digitalen Signalprozessor zur Datenverarbeitung aufweisen oder ein neuronales Netz, das auf die Erkennung von Fehlern trainiert ist.

In der Figur 2 ist schematisch ein Rotor 1 einer Pumpe dargestellt, dem eine einzelne Antriebsspule 5 stirnseitig gegenübersteht. Zwischen dem Rotor 1 und der Antriebsspule 5 liegt die Wand 4 der Pumpenkammer 2. Die Rotationsachse der Pumpe ist auch hier mit 3 bezeichnet.

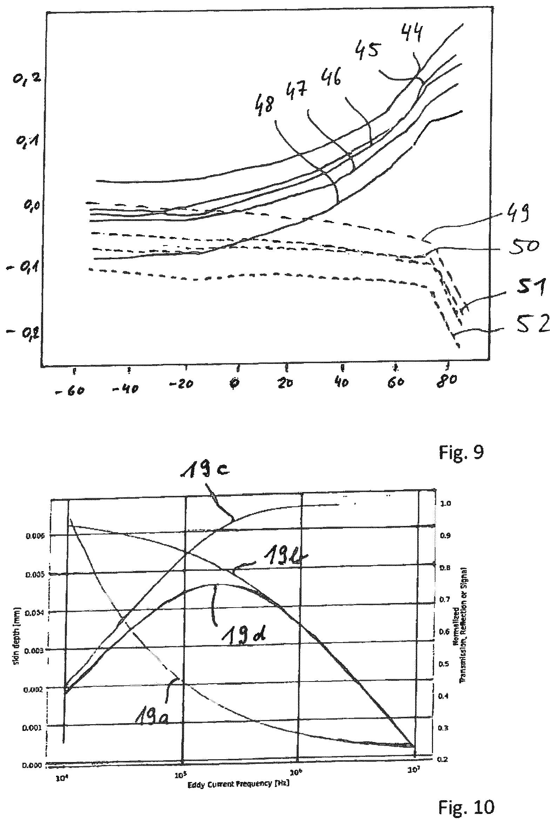

Die Antriebsspule 5 wird mit einer Mess-Wechselspannung 16 an den Anschlüssen 5b, 5c beaufschlagt, die dem Antriebsstrom überlagert ist. Der Mess- Wechselstrom wird beispielsweise über den Anschluss 17 gemessen. In der Auswerteeinheit 7a der Steuereinrichtung 7 wird aus der Mess- Wechselspannung und/oder dem Mess- Wechselstrom oder einer Kombination dieser Größen für die Frequenz der Mess-Wechselspannung eine komplexe Größe, insbesondere ein komplexer Widerstandswert ermittelt. Dieser Widerstandswert hängt unter anderem von der Wechselwirkung der Antriebsspule 5 mit dem Target 18 ab, das als flache, auf dem Rotor befestigte und mit diesem rotierende Scheibe aus einem Kupfermaterial ausgebildet ist. Insbesondere hängt der Widerstandswert von dem Abstand zwischen der Antriebsspule und dem Target ab. In dem Target 18 werden durch das Mess-Wechselmagnetfeld/das magnetische Erregungsfeld Wirbelströme erzeugt, die den Strom- und Spanungsverlauf bei der Mess-Frequenz sowie, damit verknüpft, den komplexen Widerstandswert der als Empfangsspule verwendeten Antriebsspule beeinflussen. Die Wand/Wandung 4 der Pumpenkammer besteht in dem gezeigten Beispiel aus einer Titanlegierung, in der in einem passend gewählten Frequenzbereich nur vergleichsweise geringe Wirbelströme erzeugt werden, so dass die Wand 4 für die magnetischen Wechselfelder weitgehend durchlässig ist (zu den passenden Frequenzen vgl. Figur 10). Besteht beispielsweise das Target aus Kupfer, so ist zusammen mit der Wand der Pumpenkammer aus einer Titanlegierung eine gut geeignete Materialpaarung gebildet.

In einer anderen Variante kann ein magnetisches Erregungsfeld auch durch ein anderes Element als die Antriebsspule(n) erzeugt werden, beispielsweise durch eine oder mehrere Spulen, die innerhalb der Pumpe oder an der Pumpe angeordnet sind und mit einer Mess- Wechselspannung versorgt werden können, wie beispielsweise eine Spule, die als Teil eines aktiven Magnetlagers ansteuerbar ist und als Erregungsspule fungiert. In diesem Fall werden in einer oder mehreren Antriebsspulen, die dann als Empfangsspulen dienen, Mess-Wechselspannungen und Mess-Wechselströme in einem passenden Frequenzbereich gemessen und es wird für die vorliegende Frequenz ein komplexer Widerstandswert oder eine andere Verknüpfungsgröße aus dem Mess-Wechselstrom und der Mess-Wechselspannung und eine damit korrespondierende axiale Rotorposition im Bereich der jeweiligen Empfangsspule bestimmt. Alternativ zur Bestimmung des komplexen Widerstands bei einer festen Frequenz kann auch die Frequenz eines Resonators mit dem komplexen Widerstand als frequenzbestimmendem Element als Messgröße zum Einsatz kommen.

Für die Zuordnung von Rotorpositionen in der Axialrichtung des Rotors zu den ermittelten komplexen Widerstandswerten können Eichtabellen oder eine kalibrierte digitale Umrechnungsfunktion dienen. Als Aspekte der ermittelbaren Rotorposition kommt zunächst eine Axialposition oder eine Abweichung von einer axialen Referenzposition in Frage.

Wenn die Axialposition des Rotors durch verschiedene Empfangsspulen an verschiedenen Stellen gemessen wird, die um die Rotationsachse herum verteilt sind, so lässt sich aus diesen Axialpositionen beispielsweise ein Neigungswinkel und/oder eine Radialverschiebung des Rotors und/oder ein Differenzdruck und/oder eine Durchflussrate oder eine Kombination aus diesen Größen berechnen.

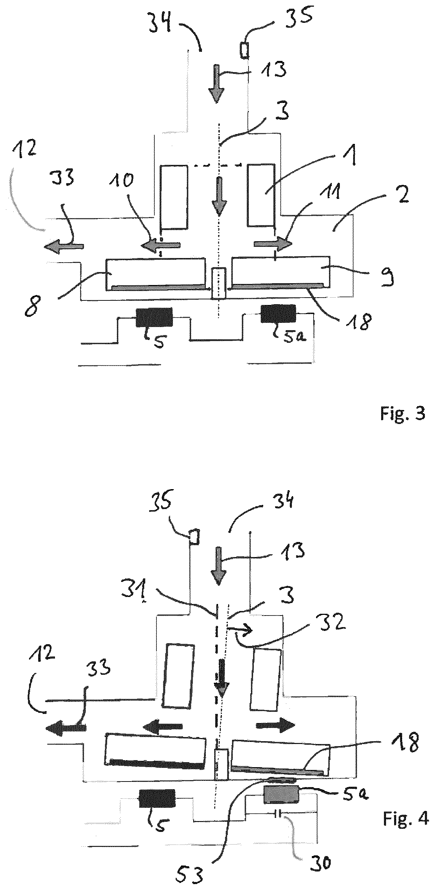

In der Figur 3 ist schematisch, ähnlich wie in der Figur 1, eine Pumpe mit einem Rotor 1 dargestellt, der eine Rotationsachse 3 aufweist. In der Ruheposition ist die Rotationsachse 3 deckungsgleich mit der Zylindersymmetrieachse 31 der Pumpe. Die zu fördernde Flüssigkeit tritt am Pumpeneinlass 34 in die Pumpe ein. An dieser Stelle kann beispielsweise ein Absolutdrucksensor 35 angeordnet sein. Vom Pumpeneinlass gelangt die Flüssigkeit, beispielsweise Blut, wie durch den Pfeil 13 angezeigt, in Axialrichtung in das Innere des Rotors 1. Der Rotor weist nicht näher dargestellte Förderelemente auf, die die zu fördernde Flüssigkeit, durch Zentrifugalkräfte radial in Richtung der Pfeile 10, 11 nach außen fördern.

Umfangsseitig weist die Pumpenkammer 2 an einer Stelle einen Pumpenauslass 12 auf, durch den die Flüssigkeit, also beispielsweise Blut, die Pumpe verlassen kann. Durch die Position des oder der Pumpenauslässe ist in vielen oder den meisten Fällen die Axialsymmetrie des Flusses in dem Pumpenraum gebrochen, so dass Kräfte entstehen, die auf den Rotor wirken und eine resultierende Radialkomponente haben.

Der Rotor 1 weist an seiner Unterseite Rotormagnete 8, 9 auf, die durch das Antriebsmagnetfeld angetrieben werden, das durch die Antriebsspulen 5, 5a erzeugt wird. Die Antriebsspulen 5, 5a können zudem als Erregungsspulen und Empfangsspulen für hochfrequente Mess-Wechselfelder dienen, um jeweils im Bereich einer Spule den Abstand zu einem im Bereich der Antriebsmagnete mit dem Rotor verbundenen Target 18 zu bestimmen. Dabei wird als Antriebsspule oft ein Spulenpaar mit zwei einzelnen Wendeln 5, 5a bezeichnet, die elektrisch in Reihe geschaltet sind und einander auf beiden Seiten der Rotationsachse/Rotorachse gegenüberliegen. Zwei solche separate Wendeln, die auch als Empfangsspulen bezeichnet werden können, bilden in dem dargestellten Beispiel ein Empfangsspulenpaar.

Bewegt sich der Rotor 1 genau in Axialrichtung 3, so weisen beide Empfangsspulen 5, 5a durch eine Änderung der Mess- Wechselspannungen und Mess-Wechselströme infolge Wechselwirkung mit dem Target 18 eine Abstandsänderung nach, die somit bestimmt werden kann.

In der Figur 4 ist eine Pumpe gemäß der Figur 3 dargestellt, wobei jedoch ein Pumpbetrieb vorausgesetzt wird, der eine Neigung des Rotors 1 erzeugt. Dies geschieht im Wesentlichen durch die zylindrische Unsymmetrie, die durch den auf einer Seite der Pumpenkammer angeordneten Pumpenauslass 12 und die dort ausströmende Flüssigkeit verursacht ist. Eine solche Unsymmetrie kann auch bei mehreren umfangsseitigen Pumpenauslässen entstehen. Durch die auftretenden resultierenden hydrodynamischen Radialkräfte wird die Rotorachse 3 gegenüber der Symmetrieachse 31 der Pumpe geneigt, was in der Darstellung der Figur 4 durch den Pfeil 32 angedeutet ist. Mit dieser Bewegung können auch noch zusätzlich eine Radialverschiebung des Rotors sowie eine Axialverschiebung auftreten.

Als Konsequenz nähert sich das Target 18 auf der Seite der Empfangsspule 5a an diese an und entfernt sich auf der Seite der Empfangsspule 5 von dieser.

Da die beiden Empfangsspulen durch Antriebsspulen gebildet sind, die elektrisch in Reihe geschaltet sind, können die Einflüsse auf die einzelnen Empfangsspulen nicht getrennt werden und kompensieren sich gegenseitig zumindest teilweise, wenn keine besonderen Maßnahmen ergriffen werden.

Gemäß der Erfindung weisen jedoch die beiden Empfangsspulen 5, 5a die Abstandsänderungen zum Target mit verschiedenen Empfindlichkeiten nach. Dadurch wird der gegenseitigen Kompensation der Messwertänderungen entgegengewirkt und eine resultierende Signaländerung erzeugt, die es erlaubt, die Rotorneigung nachzuweisen und zu messen. Die unterschiedlichen Messempfindlichkeiten der verschiedenen Empfangsspulen 5, 5a, des Empfangsspulenpaares 5, 5a werden in dem gezeigten Beispiel durch eine äußere Beschaltung der Empfangsspule 5a mit einem Kondensator 30 sowie einen asymmetrischen permeablen Materialbelag 53 an der Pumpenwand erreicht. Der Kondensator 30 steht hier nur beispielhaft für mögliche Beschaltungen, die, beispielsweise durch Ausbildung oder Abänderung eines Parallel- oder Serienschwingkreises zusammen mit der Empfangsspule 5a deren Messempfindlichkeit für eine gegebene Signalfrequenz steigern oder verringern. Es können auch beide Empfangsspulen mit unterschiedlichen Beschaltungen und/oder Permeabilitäten versehen sein. Unterbleibt eine entsprechende Beschaltung bei der jeweils anderen Empfangsspule eines Empfangsspulenpaares oder wird dort eine andere Beschaltung vorgenommen, so ergeben sich unterschiedliche Messempfindlichkeiten für die beiden Empfangsspulen bezüglich Abstandsänderungen des Targets an der jeweiligen Empfangsspule.

Wie im Folgenden gezeigt wird, besteht ein funktionaler, reproduzierbarer Zusammenhang zwischen der Rotorneigung und dem Differenzdruck, der durch die Pumpe erzeugt wird. Somit lassen sich durch eine Bestimmung des Neigungsgrades/Neigungswinkels des Rotors auch der Differenzdruck der Pumpe und/oder die Durchflussrate bestimmen. Der Differenzdruck der Pumpe und/oder die Durchflussrate lassen sich auch ohne eine Bestimmung der Rotorneigung unmittelbar aus den Messwerten der durch die Empfangsspulen gemessenen Wechselströme und/oder Wechselspannungen bestimmen.

Wird, wie anfangs erwähnt, zusätzlich am Pumpeneinlass ein Absolutdrucksensor vorgesehen, so lässt sich mit dem ermittelten Differenzdruck dann auch der Absolutdruck am Pumpenauslass ermitteln.

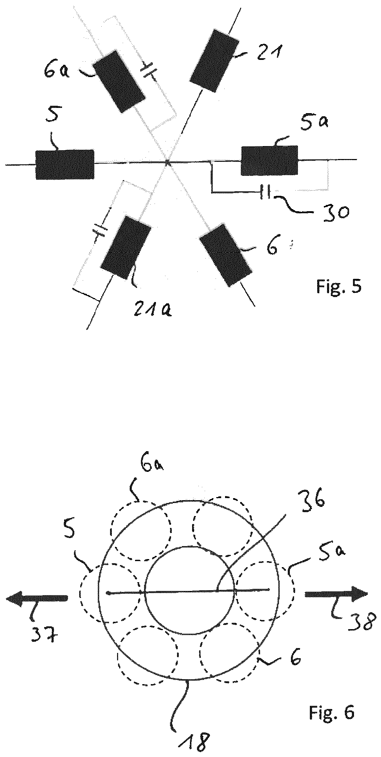

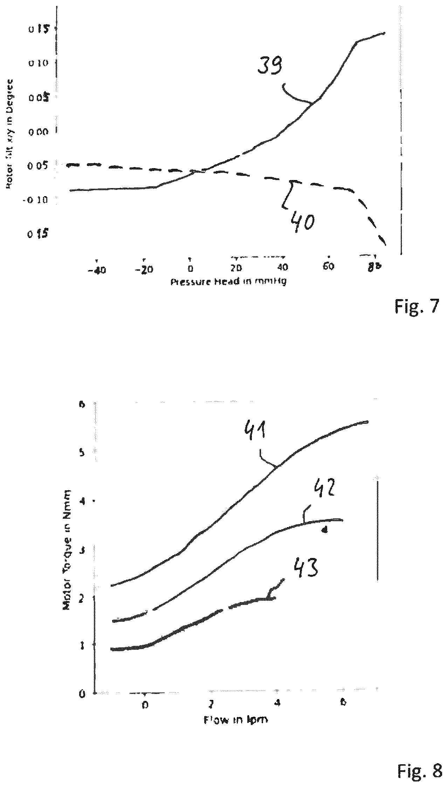

Die Figur 5 zeigt in Axialrichtung eines Pumpenrotors gesehen schematisch die Anordnung von drei Antriebsspulen, die jeweils zwei separate Teilspulen in Reihe geschaltet enthalten, wobei die Teilspulen 5, 5a, 6, 6a, 21, 21a jeweils Erregungsspulen und Empfangsspulen bilden, die zu Empfangsspulenpaaren zusammengefasst sind. Jeweils eine Empfangsspule 5a, 6a, 21a jedes Empfangsspulenpaares ist beispielshaft mit einem Kondensator 30 beschaltet, der zu der jeweiligen Empfangsspule elektrisch parallelgeschaltet ist. Jedes der Empfangsspulenpaare kann im Wesentlichen eine Neigung des Pumpenrotors in der Richtung nachweisen, in der die Verbindungslinie zwischen den beiden Empfangsspulen durch die Rotorachse hindurch verläuft. Werden zwei oder alle drei Empfangsspulenpaare zur Messung genutzt, so lässt sich die Neigung der Rotorachse im Raum insgesamt messen. Es ergeben sich dabei grundsätzlich Neigungen der Rotorachse in verschiedenen Ebenen die jeweils die Zylindersymmetrieachse der Pumpe enthalten und in dem gezeigten Beispiel jeweils um 120 Grad gegeneinander um die Rotorachse gedreht sind. Dabei kann der funktionale Zusammenhang zwischen dem Differenzdruck der Pumpe und der Rotorneigung in Richtung parallel zu einer ersten Achse strukturell anders sein als der Zusammenhang zwischen dem Differenzdruck und der Rotorneigung in der Richtung parallel zu einer anderen Achse. Dies wird weiter unten anhand des Diagramms der Figur 7 noch näher erläutert.

Zusätzlich zu einer Neigung der Rotorachse kann durch die asymmetrischen Kraftwirkungen beim Betrieb der Pumpe auch noch eine Radialverschiebung des Rotors eintreten. Auch diese kann zu Änderungen der Messwerte der Empfangsspulen führen, wie anhand der Figur 6 gezeigt werden soll. Dort ist ein Target 18 dargestellt, dessen Durchmesser so gewählt ist, dass es die Empfangsspulen 5, 5a, 6, 6a in Radialrichtung nicht vollständig überdeckt. Bei einer Verschiebung des Targets 18 in einer Radialrichtung parallel zur Verbindungsachse 36 in Richtung eines Pfeils 37, 38 wächst somit die Überdeckung des Targets mit einer der Empfangsspulen 5, 5a und gleichzeitig nimmt die Überdeckung mit der jeweils anderen Empfangsspule ab. Würden die beiden Empfangsspulen die Wechselwirkung mit dem Target im gleichen Maß/ mit der gleichen Empfindlichkeit nachweisen, so würden sich die Änderungen bei Verschiebung des Rotors näherungsweise kompensieren. Werden die Messempfindlichkeiten der beiden Empfangsspulen eines Empfangsspulenpaares unterschiedlich gestaltet, so kompensieren sich die Änderungen nicht und eine Verschiebung wird messbar.

Die Einflüsse der Rotorneigung und der radialen Rotorverschiebung sind bei der beschriebenen Gestaltung nicht immer vollständig trennbar, jedoch lassen sich durch Eichmessungen Kennlinien aufnehmen, die aus den resultierenden durch die Empfangsspulen erfassten Messwerten den Differenzdruck der Pumpe bestimmen lassen. Hierzu können gegebenenfalls noch andere erfasste Messgrößen wie die Pumpenleistung, das Rotordrehmoment, die Umdrehungszahl der Pumpe, Linearbeschleunigung und Winkelbeschleunigung der Pumpe, Messwerte eines Lagesensors der Pumpe und weitere Größen herangezogen werden. Wird das Target (18) die Antriebsspulen überdeckend ausgeführt, dann ist die Empfindlichkeit auf Radialverschiebungen signifikant reduziert und die Verkippung des Rotors kann direkt bestimmt werden. Eine Verkleinerung des Targets (5) kann auch aufgrund klinischer oder strömungstechnischer Randbedingungen notwendig sein.

In der Figur 7 ist der funktionelle Zusammenhang zwischen der Rotorneigung einer Pumpe in einer ersten Richtung, x- Richtung genannt, und in einer zweiten, y-Richtung genannten Richtung einerseits und dem Pumpendifferenzdruck andererseits dargestellt. Die x- Richtung und die y- Richtung können dabei beispielsweise einen Winkel von 90 oder 120 Grad einschließen, jedoch soll der Zusammenhang hier allgemein und ohne eine Beschränkung auf bestimmte Winkel erläutert werden.

Auf der vertikalen Achse ist dazu der Neigungswinkel des Rotors von minus 0,15 Grad bis plus 0,15 Grad aufgetragen, während auf der horizontalen Achse der Differenzdruck der Pumpe in mmHg von minus 40 mmHg bis plus 80 mmHg aufgetragen ist. Die Kurve 39 stellt dabei die Abhängigkeit des Differenzdrucks von der Neigung in x- Richtung dar und die Kurve 40 die Abhängigkeit von der Neigung in y-Richtung. Es zeigt sich, dass mit steigendem Differenzdruck die Neigung in x-Richtung steigt, während die Neigung in y-Richtung sinkt.

Im Bereich von negativen Drücken ist die Abhängigkeit nur schwach ausgeprägt. In diesem Bereich oder auch über den gesamten Messbereich könnte auch beispielsweise die Differenz der Neigungen in x- und y- Richtung zur Bestimmung des Differenzdrucks herangezogen werden, um eine höhere Empfindlichkeit zu erreichen. Die Neigungen in x- und y- Richtung können generell im gesamten Messbereich geeignet miteinander verknüpft werden, um einen großen Messbereich und eine optimierte Empfindlichkeit zu erreichen.

In der Figur 8 ist der Zusammenhang zwischen dem Drehmoment des Rotors, aufgetragen in Nmm auf der vertikalen Achse, und der auf der horizontalen Achse aufgetragenen Volumendurchflussrate, gemessen in Liter pro Minute, für drei verschiedene Umdrehungszahlen der Pumpe, nämlich 4300 rpm (Kurve 41), 3390 rpm (Kurve 42) und 4300 rpm (Kurve 43) dargestellt. Da sowohl die Umdrehungszahl/Geschwindigkeit der Pumpe, als auch das Drehmoment des Rotors, zusätzlich zu der Rotorneigung oder - Radialverschiebung messbar sind, können diese Größen auch zusätzlich zur Plausibilisierung des Differenzdrucks oder zu seiner genaueren Berechnung mit herangezogen werden.

In der Figur 9 ist für verschiedene Lagen/Orientierungen der Pumpe relativ zum Erdgravitationsfeld jeweils der funktionale Zusammenhang zwischen dem Neigungswinkel des Rotors in x-Richtung und in y-Richtung, aufgetragen auf der vertikalen Achse, und dem auf der horizontalen Achse aufgetragenen Differenzdruck der Pumpe, dargestellt. Dabei reicht die Skala der Neigungen von minus 0,2 Grad bis plus 0,2 Grad und die Differenzdrücke reichen von minus 60 mmHg bis plus 80 mmHg. In der Kurvenschar stellen die Kurven 44, 45, 46, 47, 48 die Abhängigkeit der Rotorneigung in x-Richtung vom Differenzdruck dar, während die Kurven 49, 50, 51, 52 die Abhängigkeit der Rotorneigung in y-Richtung vom Differenzdruck darstellen. Außer der Tatsache, dass die Rotorneigung in x- und y- Richtung in verschiedener Weise vom Differenzdruck abhängen zeigt sich auch, dass jeweils bei gleicher Rotorneigung der Differenzdruck wesentlich von der Orientierung der Pumpe abhängt. Aus diesem Grund ist es sinnvoll, bei der Bestimmung des Differenzdrucks die Lage der Pumpe mit einem Sensor zu messen oder dafür zu sorgen, dass bei Differenzdruckmessungen grundsätzlich eine standardisierte Pumpenorientierung sichergestellt wird. Bei implantierten Blutpumpen bedeutet dies, dass der Patient bei der Messung eine bestimmte Position einnehmen sollte.

In der Figur 10 sind für verschiedene Materialien die Wirbelstromverhältnisse in Abhängigkeit von der Frequenz der magnetischen Wechselfelder dargestellt. Dabei bezeichnet die Kurve 19a die Eindringtiefe des Stroms in dem Titanmaterial TiAIV, die sich auf-grund der Stromverdrängung durch den Skin-Effekt einstellt. Die Kurve 19b stellt den frequenzabhängigen Verlauf der Transmission eines Wechselfeldes durch das Titanmaterial dar. Die Kurve 19c zeigt den an einem Kupfertarget reflektierten Anteil des magnetischen Wechselfeldes und die Kurve 19d stellt die gemessene Signalstärke infolge der Wechselwirkung des Wechselfeldes durch eine Titanwandung hindurch mit einem Kupfertarget dar, welche das Produkt aus 19b und 19c ist.

Damit ergibt sich bei der Materialwahl von Kupfer für das Target und TiAIV für die Pumpenwandung eine gute Signalstärke im Frequenzbereich zwischen 20 kHz und 2Mhz, insbesondere zwischen 50kHz und 100kHz oder auch zwischen 50kHz und 200kHz.

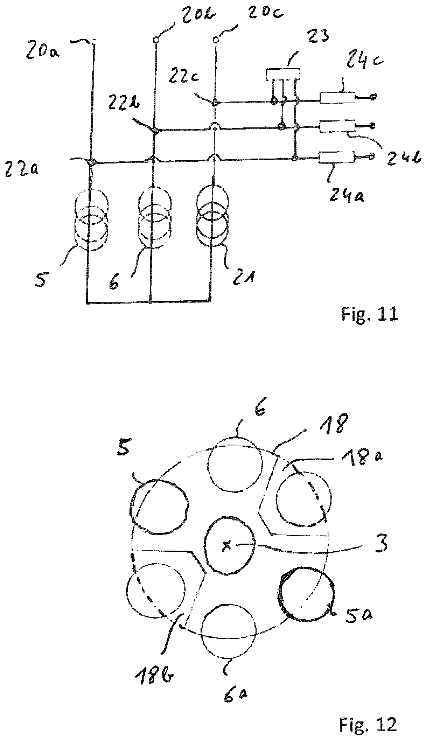

Die Figur 11 zeigt ein schematisches Phasenanschlussdiagramm eines dreiphasigen Elektromotors, der als Antrieb für eine Pumpe dient. Über die Anschlüsse 20a, 20b, 20c wird außer den Antriebsströmen in die Antriebsspulen 5, 6, 21 das Mess- Wechselspannungssignal eingespeist. Der Mess-Wechselstrom, repräsentiert durch eineSpannungs- und/oder Strommessung, wird an den Anschlüssen 22a, 22b, 22c erfasst. In einem Frequenzmischer 23 wird den gemessenen Mess-Wechselspannungssignalen ein weiteres, relativ hochfrequentes Wechselspannungssignal, beispielsweise im Bereich von 50kHz, überlagert und in einem Tiefpass 24a, 24b, 24c wird aus dem Mischsignal jeweils ein niederfrequentes Signal ausgekoppelt, das dem Verlauf der gemessenen, nur wenig zeitlich veränderlichen Mess-Wechselströme entspricht.

Aus den Signalen von Mess-Wechselspannungen und Mess-Wechselströmen lassen sich dann die komplexen Widerstandswerte der Empfangsspulen bei der Frequenz der Signale und nachfolgend die Rotorposition ermitteln.

Mischer 23 und Tiefpass 24a, 24b, 24c lassen für jede Phase in einem einzelnen analogen oder digitalen Filter vereinen. Digitale Filter, beispielsweise aufgebaut als FIR oder IIR Filter, können dabei besonders gute Filtereigenschaften aufweisen. Es können in die Tiefpassfunktion auch weitere Filterfunktionen integriert werden, um beispielsweise bekannte Störer wie eine PWM-(Pulsweitenmodulations)-Frequenz, Motorantriebsströme oder Lagerantriebsströme zu unterdrücken.

Die Figur 12 zeigt schematisch in einer stirnseitigen Ansicht, das heißt in Richtung parallel zur Rotorachse 3 eines Pumpenmotors gesehen, drei Empfangsspulenpaare, die beispielsweise durch Antriebsspulen 5, 5a, 6, 6a gebildet sein können. Dabei bilden jeweils zwei Empfangsspulen 5, 5a, 6, 6a ein Empfangsspulenpaar, dessen Empfangsspulen einander am Umfang des Stators gegenüberstehen. In dem gezeigten Beispiel stehen jeweils die Empfangsspulen 5 und 5a sowie 6 und 6a einander diametral und bezüglich der Rotorachse 3 spiegelbildlich gegenüber. Weiter ist in der Figur 6 ein kreisringscheibenförmiges Kupfertarget 18 dargestellt, das teilweise an seinem Umfang mit Ausschnitten/Ausnehmungen 18a, 18b versehen ist. Diese gelten in der Nomenklatur des vorliegenden Textes als Bereiche des Targets mit verringerter elektrischer Leitfähigkeit. Als Target wird dabei das gesamte kreisscheibenförmige Target verstanden, das durch die Ausnehmungen 18a, 18b vervollständigt ist. Die Kreisscheibenform bildet somit die Gesamtkontur des Targets. Die vervollständigte Kreisform am Umfang des Kupfertargets ist teilweise gestrichelt dargestellt, um die Ausschnitte besser sichtbar zu machen. Die beiden Ausschnitte 18a, 18b bilden Kreisringsegmente, die einander am Umfang des Kupfertargets gegenüberliegen. Der Einfluss des Kupfertargets auf den in den Empfangsspulen gemessenen komplexen Widerstand bei der Frequenz der Mess- Wechselspannung ist durch die Ausschnitte 18a, 18b stark von der Drehwinkelposition des Targets und damit des Rotors abhängig, nämlich mit der Rotationsfrequenz moduliert. Somit lässt sich mit den Empfangsspulen/Antriebsspulen unter anderem auch die Rotationsgeschwindigkeit des Rotors messen.

## Patentansprüche

1. Pumpe, insbesondere Blutpumpe, umfassend
eine Pumpenkammer (2), in der ein um eine Rotationsachse (3) drehbar gelagerter Rotor (1) mit wenigstens einem Förderelement (1a, 1b) zur Förderung von einer Flüssigkeit, insbesondere Blut, in einer Radialrichtung oder mit einer Radialrichtungskomponente angeordnet ist,
eine Wandung (4), die die Pumpenkammer umgibt, wobei die Pumpenkammer einen umfangsseitigen Pumpenauslass aufweist,
einen Stator mit mehreren Antriebsspulen (5, 6, 21) zur Erzeugung eines Antriebsmagnetfeldes, das dazu eingerichtet ist, eine Rotationsbewegung des Rotors anzutreiben sowie
eine Steuereinrichtung (7), die dazu eingerichtet ist, Antriebsströme in den Antriebsspulen für den Rotationsantrieb des Rotors zu erzeugen,
wobei der Rotor ein wenigstens teilweise elektrisch leitendes Target (18, 18') in Form eines Körpers aufweist, der von Mess-Wechselmagnetfeldern durchsetzbar ist, die durch eine Wechselmagnetfeldquelle, insbesondere eine oder mehrere Erregungsspulen (5, 6, 21) erzeugt werden, wobei die Pumpe ein oder mehrere Empfangsspulenpaare mit jeweils einander bezüglich der Rotationsachse gegenüberliegenden Empfangsspulen aufweist, in denen durch die Mess- Wechselmagnetfelder Wechselströme und/oder Wechselspannungen erzeugt werden, die von der Wechselwirkung der Empfangsspulen mit dem Target abhängig sind, wobei die Steuereinrichtung dazu eingerichtet ist, die Wechselströme und/oder Wechselspannungen in den Empfangsspulen zu messen und aus diesen komplexe Kenngrößen, insbesondere komplexe Widerstände von Empfangsspulen zu ermitteln,
**dadurch gekennzeichnet**, wenigstens ein Empfangsspulenpaar oder die Pumpe derart gestaltet ist, dass eine der Empfangsspulen den Abstand zum Target mit höherer Empfindlichkeit nachweist als die andere Empfangsspule und/oder dass im Falle der Erzeugung der Mess-Wechselmagnetfelder durch eine oder mehrere Erregungsspulen diese dazu eingerichtet sind, im Bereich einer der Empfangsspulen eines Empfangsspulenpaares ein stärkeres Mess- Wechselmagnetfeld zu erzeugen als in dem Bereich der bezüglich der Rotationsachse gegenüberliegenden Empfangsspule, und dass die Steuereinrichtung (7) dazu eingerichtet ist, aus den in einem Empfangsspulenpaar gemessenen Mess-Wechselspannungen und/oder Mess-Wechselströmen komplexe Empfangsspulen-Zustandsgrößen, insbesondere komplexe Empfangsspulen-Widerstände, des wenigstens einen Empfangsspulenpaares zu ermitteln und mit diesen Zustandsgrößen einen Differenzdruck und/oder eine Durchflussrate der Pumpe und/oder einen Neigungsgrad des Rotors und/oder eine Radialverschiebung des Rotors zu ermitteln.

2. Pumpe nach Anspruch 1, **dadurch gekennzeichnet, dass** die Steuereinrichtung dazu eingerichtet ist, den Differenzdruck und/oder eine Durchflussrate der Pumpe mit ermittelten Empfangsspulen-Zustandsgrößen des Rotors sowie einem oder mehreren der folgenden Messgrößen und/oder ihren zeitlichen Ableitungen zu ermitteln, insbesondere mittels eines trainierten selbstlernenden Systems oder eines Beobachters, der mit den Messgrößen ein die Messgrößen und mathematische Ableitungen der Messgrößen beschreibendes Differentialgleichungssystem durch ein numerisches Verfahren löst, oder eines in einer Speichereinrichtung hinterlegten Kennlinienfeldes:
Änderungsgeschwindigkeit der Empfangsspulen-Zustandsgrößen,
zweite zeitliche Ableitung der Empfangsspulen-Zustandsgrößen,
Umdrehungsgeschwindigkeit des Rotors,
Leistung des Rotorantriebs,
Stromstärke des Antriebsstroms,
Axialposition des Rotors,
Ausrichtung der Pumpe relativ zum Erdgravitationsfeld,
lineare, auf die Pumpe wirkende Beschleunigung sowie
auf die Pumpe wirkende Drehbeschleunigung.

3. Pumpe nach einem der Patentansprüche 1 oder 2, **dadurch gekennzeichnet, dass** die Pumpe am Umfang ihres Stators verteilt mehrere Empfangsspulenpaare von einander bezüglich der Rotationsachse gegenüberliegenden Empfangsspulen aufweist, und die Steuereinrichtung (7) dazu eingerichtet ist, die Empfangsspulen-Zustandsgrößen mehrerer Paare von Empfangsspulen zu ermitteln und diese insbesondere miteinander zur Ermittlung eines Differenzdrucks der Pumpe zu verknüpfen.

4. Pumpe nach einem der Ansprüche 1 bis 3, **dadurch gekennzeichnet, dass** die Pumpe am Umfang ihres Stators verteilt mehrere Paare von einander bezüglich der Rotationsachse gegenüberliegenden Empfangsspulen aufweist, und die Steuereinrichtung (7) dazu eingerichtet ist, die Empfangsspulen-Zustandsgrößen mehrerer Paare von Empfangsspulen zu ermitteln und eine Neigung und/oder Radialverschiebung des Rotors in einer ersten Ebene zu ermitteln, die die Rotorachse enthält, sowie die Neigung und/oder Radialverschiebung in einer zu dieser Ebene um die Rotorachse gedreht liegenden zweiten Ebene und dass die Steuereinrichtung weiter dazu eingerichtet ist, mit der Neigung und/oder Radialverschiebung des Rotors in der ersten Ebene oder einer Kombination der Neigungen und/oder Radialverschiebungen in der ersten Ebene und der zweiten Ebene den Differenzdruck und /oder die Durchflussrate der Pumpe zu ermitteln.

5. Pumpe nach einem der Ansprüche 1 bis 4, **dadurch gekennzeichnet, dass** sie einen Beschleunigungs- oder Lagesensor und/oder einen Absolutdrucksensor und/oder einen Magnetfeldsensor, insbesondere einen Hallsensor, aufweist.

6. Pumpe nach einem der Ansprüche 1 bis 5, **dadurch gekennzeichnet, dass** das Target als ein fest mit dem Rotor verbundener Körper, insbesondere als Scheibe oder als Ring, ausgebildet ist, der mit dem Rotor um dessen Rotationsachse rotiert und insbesondere in azimutaler Richtung bezüglich der Rotorachse betrachtet einen oder mehrere erste Bereiche mit einer ersten elektrischen Leitfähigkeit und einen oder mehrere zweite Bereiche mit einer von der ersten elektrischen Leitfähigkeit verschiedenen zweiten elektrischen Leitfähigkeit aufweist.

7. Pumpe nach einem der Ansprüche 1 bis 6, **dadurch gekennzeichnet, dass** das Target (18) die Form einer ebenen, mit dem Rotor fest verbundenen Scheibe aufweist, deren Flächennormale in Richtung zu einer oder mehreren Empfangsspulen orientiert ist und dass der durch die Antriebsspulen (5, 6, 21) erzeugte magnetische Fluss des Motors im Target überwiegend in Axialrichtung (3) des Rotors verläuft oder dass das Target die Form eines mit dem Rotor fest verbundenen, am äußeren Umfang des Rotors angeordneten Torus aufweist und dass der durch die am Umfang des Pumpengehäuses angeordneten Antriebsspulen erzeugte magnetische Fluss des Motors im Target überwiegend in Radialrichtung des Rotors verläuft.

8. Pumpe nach einem der Ansprüche 1 bis 7, **dadurch gekennzeichnet, dass** das Target (18) die Empfangsspulen eines Empfangsspulenpaares nicht vollständig überdeckt oder wenigstens während des Betriebs der Pumpe nicht vollständig überdeckt.

9. Pumpe nach einem der Ansprüche 1 bis 8, **dadurch gekennzeichnet, dass** die Empfangsspulenpaare (5, 6, 21) auf der Außenseite der Wandung (4) der Pumpenkammer (2) angeordnet sind und dass die Wandung der Pumpenkammer mehr als 25% des Mess-Wechselmagnetfeldes transmittiert und das Target mehr als 25% des Mess-Wechselmagnetfeldes reflektiert oder absorbiert und/oder dass die Wandung (4) der Pumpenkammer aus einem ersten Material und das Target (18, 18') überwiegend aus einem zweiten Material besteht, wobei das zweite Material bei der Frequenz der Mess-Wechselspannung einen geringeren elektrischen Widerstand aufweist als das erste Material und wobei insbesondere das erste Material Titan oder eine Titanlegierung ist und/oder das zweite Material Kupfer oder eine Kupferlegierung.

10. Pumpe nach einem der Ansprüche 1 bis 9, **dadurch gekennzeichnet, dass** die Steuereinrichtung (7) dazu eingerichtet ist, in einer oder mehreren der Erregungsspulen (5, 6, 21) jeweils periodische Mess-Wechselspannungen mit einer Frequenz zwischen 20kHz und 2MHz, insbesondere zwischen 50kHz und 100 kHz, zu erzeugen.

11. Pumpe nach einem der Ansprüche 1 bis 10, **dadurch gekennzeichnet, dass** eine, mehrere oder alle Empfangsspulen und/oder Erregungsspulen mit Antriebsspulen des Stators der Pumpe identisch oder mit diesen elektrisch verbunden sind.

12. Pumpe nach einem der Ansprüche 1 bis 11, **dadurch gekennzeichnet, dass** jeweils eine der Empfangsspulen eines Empfangsspulenpaares , insbesondere im Fall, dass die Empfangsspulen mit Antriebsspulen des Stators der Pumpe identisch sind, eine der Antriebsspulen eines Paares, mit einem oder mehreren Elementen eines Schwingkreises, insbesondere mit einem Kondensator, verbunden ist und/oder dass in den beiden Empfangsspulen eines Empfangsspulenpaares oder in deren Umgebung Elemente mit verschiedener magnetischer Permeabilität angeordnet sind.

13. Verfahren zur Bestimmung des Differenzdrucks einer Pumpe, insbesondere einer Blutpumpe, die
eine Pumpenkammer (2), in der ein um eine Rotationsachse (3) drehbar gelagerter Rotor (1) mit wenigstens einem Förderelement (1a, 1b) zur Förderung von einer Flüssigkeit, insbesondere Blut, in einer Radialrichtung oder in einer Richtung mit einer Radialrichtungskomponente angeordnet ist,
eine Wandung (4), die die Pumpenkammer umgibt, wobei die Pumpenkammer einen umfangsseitigen Pumpenauslass aufweist,
einen Stator mit mehreren Antriebsspulen (5, 6, 21) zur Erzeugung eines Antriebsmagnetfeldes, das dazu eingerichtet ist, eine Rotationsbewegung des Rotors anzutreiben sowie
eine Steuereinrichtung (7) umfasst, die dazu eingerichtet ist, Antriebsströme in den Antriebsspulen für den Rotationsantrieb des Rotors zu erzeugen, **dadurch gekennzeichnet, dass** während des Betriebs der Pumpe eine Neigung und/oder Radialverschiebung der Rotorachse oder eine von diesen abhängige elektrische Größe und/oder zeitliche Ableitungen dieser Größen ermittelt wird/werden und mit einer oder mehreren dieser Größen ein Differenzdruck und/oder eine Durchflussrate der Pumpe ermittelt wird.

14. Verfahren nach Anspruch 13, **dadurch gekennzeichnet, dass** während der Ermittlung der Neigung und/oder Radialverschiebung der Rotorachse oder einer von diesen abhängigen elektrischen Größe mittels eines Lagesensors die Lage der Pumpe relativ zum Gravitationsfeld der Erde und insbesondere eine lineare Beschleunigung und/oder eine Winkelbeschleunigung der Pumpe ermittelt und bei der Ermittlung des Differenzdrucks und/oder der Durchflussrate als Korrekturgröße berücksichtigt wird/werden.
